(19) Europäisches Patentamt
European Patent Office
Office européen des brevets

(11)  EP 2 672 952 B1

(12)  **EUROPEAN PATENT SPECIFICATION**

(45) Date of publication and mention
of the grant of the patent:
**15.06.2016  Bulletin 2016/24**

(51) Int Cl.:
*A61K 8/81* (2006.01)       *A61Q 5/06* (2006.01)
*A61Q 5/12* (2006.01)       *A61Q 5/00* (2006.01)

(21) Application number: **12703291.0**

(22) Date of filing: **03.02.2012**

(86) International application number:
**PCT/EP2012/051893**

(87) International publication number:
**WO 2012/107368 (16.08.2012 Gazette 2012/33)**

(54) **HAIR TREATMENT COMPOSITIONS**

ZUSAMMENSETZUNG ZUR HAARBEHANDLUNG

COMPOSITION DE TRAITEMENT CAPILLAIRE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **09.02.2011  EP 11153795**

(43) Date of publication of application:
**18.12.2013  Bulletin 2013/51**

(73) Proprietors:
• **Unilever PLC**
**London, Greater London EC4Y 0DY (GB)**
Designated Contracting States:
**CY GB IE MT**
• **Unilever N.V.**
**3013 AL Rotterdam (NL)**
Designated Contracting States:
**AL AT BE BG CH CZ DE DK EE ES FI FR GR HR
HU IS IT LI LT LU LV MC MK NL NO PL PT RO RS
SE SI SK SM TR**

(72) Inventors:
• **AKINPELU, Akinwole, Oladiran**
**Wirral**
**Merseyside**
**CH63 3JW (GB)**
• **EKANI NKODO, Axel, Herve**
**Shanghai 20335 (CN)**
• **PAUL, Prem, Kumar, Cheyalazhagan**
**Wirral**
**Merseyside**
**CH63 3JW (GB)**

(74) Representative: **James, Helen Sarah**
**Unilever PLC**
**Unilever Patent Group**
**Colworth House, Sharnbrook**
**Bedford**
**Bedfordshire MK44 1LQ (GB)**

(56) References cited:
**WO-A1-2007/071308      US-A1- 2001 022 967**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

This invention relates to hair treatment compositions and to their use in the treatment of hair.

Pressure sensitive adhesives (PSAs) have been used in hair care compositions as described in US5166276, EP408311, EP412707 and EP412704. However these PSAs tend to hydrolyse in aqueous and hydroalcoholic hair care products.

WO2007/071308 discloses compositions comprising pressure sensitive adhesives having superior foaming properties.

The present invention concerns hair care compositions with improved re-styling benefits.

## Description of the Invention

Accordingly, this invention provides a hair treatment composition comprising an acrylic pressure sensitive adhesive consisting of a 98:2 wt ratio copolymer of butyl acrylate and methacrylic acid.

A method for styling hair is also described which comprises contacting the hair with the composition described above.

Use of the PSA described above to re-style hair is also disclosed.

## Detailed Description of the Invention

Unless specified otherwise, all wt% values quoted hereinafter are percentages by weight based on total weight of the hair treatment composition.

## Silicone Pressure Sensitive Adhesives

This present invention relates to hair treatment compositions comprising an acrylic pressure sensitive adhesive.

"Pressure sensitive adhesive" (PSA) materials are permanently tacky at room temperature and able to develop measurable adhesion to a surface simply upon contact or by the application of a light pressure. Generally they do not require heat. No chemical reaction takes place between the adhesive and the adherent, no curing of the adhesive is necessary and no solvent is required to be lost during the adhesion process.

The acrylic PSAs of the invention consist of a 98:2 wt ratio copolymer of butyl acrylate and methacrylic acid.

Small molecule additives such as tackifiers may be included, essentially to adjust the Tg and optimise dissipative properties but are not essential.

The acrylic sensitive pressure adhesive is preferably in the form of an emulsion.

Water-born acrylic sensitive pressure adhesive is Roderm MD-5800 ex Rohm and Haas.

Preferably the acrylic pressure sensitive adhesive is present at levels from 0.01% to 10% by weight of the total composition. More preferred amounts of acrylic pressure sensitive adhesive in the compositions of the invention are from 0.1% to 5% by weight of the composition, even more preferably from 0.5% to 3.5% by weight.

## Hair Styling Polymer

The compositions of the invention may optionally comprise from 0.001% to 10% by weight of a hair styling polymer.

More preferred amounts of hair styling polymer in the compositions of the invention are from 0.1 % to 5% by weight of the composition, even more preferably from 0.5% to 3% by weight.

Hair styling polymers are well known. Suitable hair styling polymers include commercially available polymers that contain moieties that render the polymers cationic, anionic, amphoteric or nonionic in nature. Suitable hair styling polymers include, for example, block and graft copolymers. The polymers may be synthetic or naturally derived.

## Surfactant

The compositions of the invention may comprise surfactant in addition to that required for the preparation of any PSA emulsion. The surfactants which are suitable for use in the compositions of the invention may be nonionic, cationic, anionic, zwitterionic or a mixture of such surfactants depending on the product form.

The hair styling compositions of the invention preferably comprise a non-ionic surfactant, in an amount of up to 5%, preferably from 0.01% to 1%, most preferably from 0.02% to 0.8% by weight based on total weight.

Examples of suitable non-ionic surfactants are condensation products of aliphatic ($C_8$-$C_{18}$) primary or secondary linear or branched chain alcohols or phenols with alkylene oxides, usually ethylene oxide and generally having at least 15, preferably at least 20, most preferably from 30 to 50 ethylene oxide groups. Other suitable non-ionics include esters of sorbitol, esters of sorbitan anhydrides, esters of propylene glycol, fatty acid esters of polyethylene glycol, fatty acid esters of polypropylene glycol, ethoxylated esters and polyoxyethylene fatty ether phosphates.

Of particular use are those non-ionic surfactants of general formula $R(EO)_x H$, where R represents a straight or branched chain alkyl group having an average carbon chain length of 12-18 carbon atoms and x ranges from 30 to 50. Specific examples include steareth-40, steareth-50, ceteareth-30, ceteareth-40, ceteareth-50 and mixtures thereof. Suitable commercially available examples of these materials include Unicol SA-40 (Universal Preserv-A-Chem), Empilan KM50 (Albright and Wilson), NONION PS-250 (Nippon Oils & Fats), Volpo CS50 (Croda Inc), and Incropol CS-50 (Croda Inc).

## Water

Compositions of the present invention can also include water, preferably distilled or de-ionised, as a carrier for the PSAs. Water is preferably used as an emulsion when used as a carrier for acrylic PSAs. It may additionally be a carrier or a solvent for other components. When present the water will typically be present in amounts ranging from 30% to 98%, preferably from 50% to 95% by weight.

## Solvent/Carrier

Compositions of the present invention can also include solvents, as a carrier or solvent for the acrylic PSAs and other components. When present the solvent will typically be present in amounts ranging from 30% to 98%, preferably from 50% to 95% by weight. Examples of solvents are hydrocarbons, esters, alcohols etc. Particularly preferred solvents include ethyl acetate and isopropanol.

## Emollients

Emollients such as hydrocarbons, esters, silicone fluids, may be included in the compositions of the invention. Emollients may typically be present in compositions of the invention in amounts of from 0.001% to 10% by weight, preferably 0.1 % to 3% by weight. Emollients may be single compounds or mixtures of two or more compounds from the same class or different general classes.

Emollients may be included in any of the compositions of the invention, regardless of whether they contain a hair styling polymer. In one embodiment of the invention, the compositions (such as aerosol mousse formulations, for example) comprise a hair conditioning agent and are substantially free of hair styling polymer.

Suitable hydrocarbons can be either straight or branched chain and can contain from about 10 to about 16, preferably from about 12 to about 16 carbon atoms. Examples of suitable hydrocarbons are decane, dodecane, tetradecane, tridecane, and mixtures thereof.

Suitable oily or fatty materials are selected from hydrocarbon oils, fatty esters and mixtures thereof.

Straight chain hydrocarbon oils will preferably contain from about 12 to about 30 carbon atoms. Also suitable are polymeric hydrocarbons of alkenyl monomers, such as $C_2$-$C_6$ alkenyl monomers.

Specific examples of suitable hydrocarbon oils include paraffin oil, mineral oil, saturated and unsaturated dodecane, saturated and unsaturated tridecane, saturated and unsaturated tetradecane, saturated and unsaturated pentadecane, saturated and unsaturated hexadecane, and mixtures thereof. Branched-chain isomers of these compounds, as well as of higher chain length hydrocarbons, can also be used.

Suitable fatty esters are characterised by having at least 10 carbon atoms, and include esters with hydrocarbyl chains derived from fatty acids or alcohols, Monocarboxylic acid esters include esters of alcohols and/or acids of the formula R'COOR in which R' and R independently denote alkyl or alkenyl radicals and the sum of carbon atoms in R' and R is at least 10, preferably at least 20. Di- and trialkyl and alkenyl esters of carboxylic acids can also be used.

Particularly preferred fatty esters are mono-, di- and triglycerides, more specifically the mono-, di-, and tri-esters of glycerol and long chain carboxylic acids such as $C_1$-$C_{22}$ carboxylic acids. Preferred materials include cocoa butter, palm stearin, sunflower oil, soyabean oil and coconut oil.

The oily/fatty material is suitably present at a level of from 0.05 to 10, preferably from 0.2 to 5, more preferably from about 0.5 to 3 wt%.

Examples of suitable silicone based emollients useful herein can include either cyclic or linear polydimethylsiloxanes, phenyl and alkyl phenyl silicones, and silicone copolyols. Cationic conditioning agents useful herein can include quaternary ammonium salts or the salts of fatty amines, such as cetyl ammonium chloride, for example.

Compositions according to the invention may, optionally, comprise from 0.1% to 10% by weight of a volatile silicone as the hair conditioning agent. Volatile silicones are well known in the art and are commercially available and include, for example linear and cyclic compounds. Volatile silicone oils are preferably linear or cyclic polydimethylsiloxanes containing from about three to about nine silicon atoms.

The compositions of the invention may optionally comprise a cross-linked silicone polymer.

The cross-linked silicone polymer is preferably a non-rigid emulsion-polymerised and may be present in compositions of the invention in an amount of up to 10% by weight based on the total weight of the composition, more preferably from 0.2% to 6% by weight, most preferably from 0.5 to 5% by weight.

Preferred silicone polymers for use in the invention are polydiorganosiloxanes, preferably derived from suitable combinations of $R_3SiO_{0.5}$ units and $R_2SiO$ units where each R independently represents an alkyl, alkenyl (e.g., vinyl), alkaryl, aralkyl, or aryl (e.g. phenyl) group. R is most preferably methyl.

The preferred silicone polymers of the invention are cross-linked polydimethyl siloxanes (which have the CTFA designation dimethicone), and cross-linked polydimethyl siloxanes having end groups such as hydroxyl (which have the CTFA designation dimethiconol). Good results have been obtained with cross-linked dimethiconol.

Cross-linking of the silicone polymer is typically introduced concurrently during emulsion polymerisation of the polymer through the inclusion of the required amount of trifunctional and tetrafunctional silane monomer units, for example, those of formula:

R Si (OH)$_3$ wherein R represents an alkyl, alkenyl (e.g. vinyl), alkaryl, aralkyl or aryl (e.g. phenyl) group, preferably methyl.

The degree of cross-linking of the silicone polymer can be measured as the percentage of branched monomer units in the silicone polymer and is from 0.05% to 10%, preferably being in the range 0.15% to 7%, e.g. from 0.2% to 2%. Increasing cross-linking is found to improve styling benefits but also to reduce conditioning performance somewhat, so compromise levels must be selected with properties optimised to suit consumer preferences in different cases. Good overall performance has been obtained with dimethiconol 0.3% cross-linked.

Suitable emulsion polymerised cross-linked silicone polymers are commercially available or can be readily made using conventional techniques well known to those skilled in the art.

Cross-linked silicone polymers are described in EP 818190, the contents of which are incorporated herein by reference.

**Cationic Conditioning Agents**

The compositions of the invention may optionally comprise cationic conditioning agents, in particular cationic surfactants, used singly or in admixture.

Cationic surfactants useful in compositions of the invention contain amino or quaternary ammonium hydrophilic moieties, which are positively charged when, dissolved in the aqueous composition of the present invention.

Examples of suitable cationic surfactants are those corresponding to the formula:

$$[N(R_1)(R_2)(R_3)(R_4)]^+ (X)^-$$

in which $R_1$, $R_2$, $R_3$, and $R_4$ are independently selected from (a) an aliphatic group of from 1 to 22 carbon atoms, or (b) an aromatic, alkoxy, polyoxyalkylene, alkylamido, hydroxyalkyl, aryl or alkylaryl group having up to 22 carbon atoms; and X is a salt-forming anion such as those selected from halogen, (e.g. chloride, bromide), acetate, citrate, lactate, glycolate, phosphate nitrate, sulphate, and alkylsulphate radicals.

The aliphatic groups can contain, in addition to carbon and hydrogen atoms, ether linkages, and other groups such as amino groups. The longer chain aliphatic groups, e.g., those of about 12 carbons, or higher, can be saturated or unsaturated.

The most preferred cationic surfactants for conditioner compositions of the present invention are monoalkyl quaternary ammonium compounds in which the alkyl chain length is C8 to C14.

Suitable examples of such materials correspond to the formula:

$$[N(R_5)(R_6)(R_7)(R_8)^+ (X)^-$$

in which $R_5$ is a hydrocarbyl chain having 8 to 14 carbon atoms or a functionalized hydrocarbyl chain with 8 to 14 carbon atoms and containing ether, ester, amido or amino moieties present as substituents or as linkages in the radical chain, and $R_6$, $R_7$ and $R_8$ are independently selected from (a) hydrocarbyl chains of from 1 to about 4 carbon atoms, or (b) functionalized hydrocarbyl chains having from 1 to about 4 carbon atoms and containing one or more aromatic, ether, ester, amido or amino moieties present as substituents or as linkages in the radical chain, and X is a salt-forming anion such as those selected from halogen, (e.g. chloride, bromide), acetate, citrate, lactate, glycolate, phosphate nitrate, sulphate, and alkylsulphate radicals.

The functionalised hydrocarbyl chains (b) may suitably contain one or more hydrophilic moieties selected from alkoxy (preferably $C_1$-$C_3$ alkoxy), polyoxyalkylene (preferably $C_1$-$C_3$ polyoxyalkylene), alkylamido, hydroxyalkyl, alkylester, and combinations thereof.

Preferably the hydrocarbyl chains $R_1$ have 12 to 14 carbon atoms, most preferably 12 carbon atoms. They may be derived from source oils which contain substantial amounts of fatty acids having the desired hydrocarbyl chain length. For example, the fatty acids from palm kernel oil or coconut oil can be used as a source of C8 to C12 hydrocarbyl chains.

Typical monoalkyl quaternary ammonium compounds of the above general formula for use in shampoo compositions of the invention include:

(i) lauryl trimethylammonium chloride(available commercially as Arquad C35 ex-Akzo); cocodimethyl benzyl ammonium chloride (available commercially as Arquad DMCB-80 ex-Akzo)

(ii) compounds of the formula:

$$[N(R_1)(R_2)((CH_2\,CH_2\,O)_x\,H)((CH_2\,CH_2O)_y H)]^+\,(X)^-$$

wherein:

$x + y$ is an integer from 2 to 20;

$R_1$ is a hydrocarbyl chain having 8 to 14, preferably 12 to 14, most preferably 12 carbon atoms or a functionalised hydrocarbyl chain with 8 to 14, preferably 12 to 14, most preferably 12 carbon atoms and containing ether, ester, amido or amino moieties present as substituents or as linkages in the radical chain;

$R_2$ is a $C_1$-$C_3$ alkyl group or benzyl group, preferably methyl, and

X is a salt-forming anion such as those selected from halogen, (e.g. chloride, bromide), acetate, citrate, lactate, glycolate, phosphate nitrate, sulphate, methosulphate and alkylsulphate radicals.

Suitable examples are PEG-n lauryl ammonium chlorides (where n is the PEG chain length), such as PEG-2 cocomonium chloride (available commercially as Ethoquad C12 ex-Akzo Nobel); PEG-2 cocobenzyl ammonium chloride (available commercially as Ethoquad CB/12 ex-Akzo Nobel); PEG-5 cocomonium methosulphate (available commercially as Rewoquat CPEM ex-Rewo); PEG-15 cocomonium chloride (available commercially as Ethoquad C/25 ex-Akzo)

(iii) compounds of the formula:

$$[N(R_1)(R_2)(R_3)((CH_2)_nOH)]^+(X)^-$$

wherein:

n is an integer from 1 to 4, preferably 2;

$R_1$ is a hydrocarbyl chain having 8 to 14, preferably 12 to 14, most preferably 12 carbon atoms;

$R_2$ and $R_3$ are independently selected from $C_1$ - $C_3$ alkyl groups, and are preferably methyl, and

$X^-$ is a salt-forming anion such as those selected from halogen, (e.g. chloride, bromide), acetate, citrate, lactate, glycolate, phosphate nitrate, sulphate, and alkylsulphate radicals.

Suitable examples are lauryldimethylhydroxyethylammonium chloride (available commercially as Prapagen HY ex-Clariant).

Mixtures of any of the foregoing cationic surfactants compounds may also be suitable.

Examples of suitable cationic surfactants include:

quaternary ammonium chlorides, e.g. alkyltrimethylammonium chlorides wherein the alkyl group has from about 8 to 22 carbon atoms, for example octyltrimethylammonium chloride, dodecyltrimethylammonium chloride, hexadecyltrimethylammonium chloride, cetyltrimethylammonium chloride, octyldimethylbenzylammonium chloride, decyldimethylbenzylammonium chloride, stearyldi-methylbenzylammonium chloride, didodecyldimethylammonium chloride, dioctadecyldimethylammonium chloride, tallow trimethylammonium chloride, cocotrimethylammonium chloride, and the corresponding salts thereof, e.g., bromides, hydroxides. Cetylpyridinium chloride or salts thereof, e.g., chloride Quaternium -5

Quaternium -31

Quaternium -18

and mixtures thereof.

In the conditioners of the invention, the level of cationic surfactant is preferably from 0.01 to 10, more preferably 0.05 to 5, most preferably 0.1 to 2 wt% of the total composition.

**Product Form**

Compositions of the present invention are formulated into hair styling compositions which may take a variety of forms, including, for example, mousses, gels, lotions, creams, sprays and tonics. These product forms are well known in the art.

The preferred product is a cream, spray, aerosol mousse or gel.

Aerosol-form compositions of the invention will include an aerosol propellant which serves to expel the other materials from the container, and forms the mousse character in mousse compositions. The aerosol propellant included in styling compositions of the present invention can be any liquefiable gas conventionally used for aerosol containers. Examples of suitable propellants include dimethyl ether and hydrocarbon propellants such as propane, n-butane and iso-butane. The propellants may be used singly or admixed. Water insoluble propellants, especially hydrocarbons, are preferred because they form emulsion droplets on agitation and can create suitable mousse foam densities when needed.

The amount of the propellant used is governed by normal factors well known in the aerosol art. For mousses the level of propellant is generally up to 35%, preferably from 2% to 30%, most preferably from 3% to 15% by weight based on total weight of the composition. If a propellant such as dimethyl ether includes a vapour pressure suppressant (e.g. trichloroethane or dichloromethane), for weight percentage calculations, the amount of suppressant is included as part of the propellant. For aerosol sprays the levels of propellant are usually higher; preferably from 30 to 98 wt% of the total composition, more preferably 50 to 95 wt %.

Preferred propellants are selected from propane, n-butane, isobutane, dimethyl ether and mixtures thereof. Preferably, the propellant comprises dimethyl ether and at least one of propane, n-butane and isobutane.

The method of preparing aerosol hair styling mousse compositions according to the invention follows conventional aerosol filling procedures. The composition ingredients (not including the propellant) are charged into a suitable pressurisable container which is sealed and then charged with the propellant according to conventional techniques.

Compositions of the invention may also take a non-foaming product form, such as a hair styling cream or gel. Such a cream or gel will include a structurant or thickener, typically at a level of from 0.1 % to 10%, preferably 0.5% to 3% by weight based on total weight.

Examples of suitable structurants or thickeners are polymeric thickeners such as carboxyvinyl polymers. A carboxyvinyl polymer is an interpolymer of a monomeric mixture comprising a monomeric olefinically unsaturated carboxylic acid, and from about 0.01 % to about 10% by weight of the total monomers of a polyether of a polyhydric alcohol. Carboxyvinyl polymers are substantially insoluble in liquid, volatile organic hydrocarbons and are dimensionally stable on exposure to air. Suitably the molecular weight of the carboxyvinyl polymer is at least 750,000, preferably at least 1,250,000, most preferably at least 3,000,000. Preferred carboxyvinyl polymers are copolymers of acrylic acid cross-linked with allylsucrose or allylpentaerythritol as described in US Patent 2,798,053. These polymers are provided by B.F.Goodrich Company as, for example, CARBOPOL 934, 940, 941 and 980. Other materials that can also be used as structurants or thickeners include those that can impart a gel-like viscosity to the composition, such as water soluble or colloidally water soluble polymers like cellulose ethers (e.g. methylcellulose, hydroxyethylcellulose, hydroxypropylmethylcellulose and carboxymethylcellulose), guar gum, sodium alginate, gum arabic, xanthan gum, polyvinyl alcohol, polyvinyl pyrrolidone, hydroxypropyl guar gum, starch and starch derivatives, and other thickeners, viscosity modifiers, gelling agents, etc. It is also possible to use inorganic thickeners such as bentonite or laponite clays.

The hair styling compositions of the invention can contain a variety of non-essential, optional components suitable for rendering the compositions more aesthetically acceptable or to aid use, including discharge from the container, of the product. Such conventional optional ingredients are well known to those skilled in the art, e.g. preservatives such as benzyl alcohol, methyl paraben, propyl paraben and imidazolidinyl urea, fatty alcohols such as cetearyl alcohol, cetyl alcohol and stearyl alcohol, pH adjusting agents such as citric acid, succinic acid, sodium hydroxide and triethanolamine, colouring agents such as any of the FD&C or D&C dyes, perfume oils, chelating agents such as ethylenediamine tetraacetic acid, and polymer plasticising agents such as glycerin and propylene glycol.

The invention will now be further illustrated by the following, non-limiting Examples.

Examples of the invention are illustrated by a number, comparative examples are illustrated by a letter.

Experiment 1

Dark brown wavy switches (25cm and 2gm in weigh) were washed and conditioned in Sunsilk (Lively clean and fresh). The wet switches were combed through and 0.35gm of aqueous solution containing the treatment polymer was applied to each switch and massaged through. The switches were ironed straight and were placed in an image analysis chamber at 30C and 80%RH for a further 30 minutes and agitated. The volumes of the switches were captured at initial and after the 30 minute time point. Due to the high humidity and agitation the switches would have fluffed up - displaying a loss of style. Subsequently the switches were restyled by combing 5 times and squeezing between fingers and thumb and running over the switch 5 times. The volume after restyling was also captured.

If V0 is the initial straight ironed volume, V1 the volume after 30 minutes of high humidity and agitation and V2 the volume after restyling, then

$$\text{Restyling index} = (V1-V0)*100/(V1-V2).$$

The restyling index the tested polymers are given below

**Table 1**

| Example | | Restylability Index (%) | Error at 95% confidence |
|---------|--------------------|-------------------------|--------------------------|
| B | water | 11 | 11 |
| 1 | 1% MD5800 | 27 | 10 |
| 2 | 3% MD5800 | 60 | 13 |
| C | 3% MQ Resin | 31 | 12 |
| D | 3% Luviquat Supreme | 20 | 15 |
| E | 3% PS83D | 18 | 23 |

MD5800 is a 98:2 wt ratio copolymer of butyl acrylate and methacrylic acid.

PS 83D is a 98:1.8 wt ratio copolymer of butyl acrylate and methacrylic acid

MQ resin is DC-CF0410 which is Cyclopentasiloxane and Trimethylsiloxysilicate and Dimethiconol (Dow Corning)

Luviquat Supreme is polyquarternium-68

MD5800 gives increased restyling properties compared with other pressure sensitive adhesives at the same levels

**Example 3**

The following is an Example of the invention.

| Trade Name | Chemical (INCI) Name | Supplier | Wt% |
|------------|---------------------|----------|-----|
| Roderm MD5800 | 98% ButylAcrylate (BA), 2% MethylAcrylic Acid (MAA) | Rohm & Haas | 0.5 |
| Purac HS88 | Lactic Acid | PURAC | 0.07 |
| Lexamine S-13 | Stearamidopropyl Dimethylamine | INNOLEX | 0.50 |
| Hydrenol MY | Cetearyl Alcohol | COGNIS | 1.50 |
| Tinovis CD | Dimethylacrylamide/Ethyltrimo nium Chloride Methacrylate Copolymer and Propylene Glycol Dicaprylate/Dicaprate and PPG-1 Trideceth-6 and C10-11 Isoparaffin | CIBA | 0.70 |
| Wacker Belsil HL999 | Dimethicone (and) Trideceth-5 | WACKER | 1.67 |

(continued)

| Trade Name | Chemical (INCI) Name | Supplier | Wt% |
|---|---|---|---|
| CarnationWhite Mineral Oil SU70 | Paraffinium Liquidium | SONNEBORN | 1.00 |
| Glycerin | Glycerine | UNICHEMA | 1.00 |
| DC245 | Cyclopentasiloxane | Dow Corning | 0.14 |
| Nipagin M | Methylparaben | CLARIANT | 0.20 |
| Glydant Ltd | DMDM Hydantion | LONZA | 0.30 |
|  | Perfume |  | 0.40 |
| Aqua | Aqua |  | To 100% |

## Claims

1. A hair treatment composition comprising an acrylic pressure sensitive adhesive consisting of a 98:2 wt ratio copolymer of butyl acrylate and methacrylic acid.

2. A composition according to claim 1 which further comprises a cationic conditioning surfactant.

3. A composition according to any preceding claim which further comprises an emollient.

4. A composition according to any preceding claim which further comprises a volatile silicone.

5. A composition according to any preceding claim which is a leave on composition.

6. A hair treatment composition according to any preceding claim which further comprises hydrocarbon propellant.

7. A method for styling hair which comprises contacting the hair with a composition in accordance with any preceding claim.

8. Use of an acrylic pressure sensitive adhesive consisting of a 98:2 wt ratio copolymer of butyl acrylate and methacrylic acid to re-style hair.

## Patentansprüche

1. Zusammensetzung zur Haarbehandlung, umfassend einen druckempfindlichen Acryl-Klebstoff, der aus einem Copolymer von Butylacrylat und Methacrylsäure eines 98:2 Gew.-Verhältnisses besteht.

2. Zusammensetzung nach Anspruch 1, die des Weiteren ein kationisches Konditioniertensid umfasst.

3. Zusammensetzung nach irgendeinem vorhergehenden Anspruch, die des Weiteren einen Weichmacher umfasst.

4. Zusammensetzung nach irgendeinem vorhergehenden Anspruch, die des Weiteren ein flüchtiges Silikon umfasst.

5. Zusammensetzung nach irgendeinem vorhergehenden Anspruch, die eine Verbleib-Zusammensetzung ist.

6. Zusammensetzung zur Haarbehandlung nach irgendeinem vorhergehenden Anspruch, die des Weiteren Kohlenwasserstoff-Treibmittel umfasst.

7. Verfahren zum Haarstyling, welches das Inkontaktbringen des Haars mit einer Zusammensetzung gemäß irgendeinem vorhergehenden Anspruch umfasst.

**8.** Verwendung eines druckempfindlichen Acryl-Klebstoffs, bestehend aus einem Copolymer von Butylacrylat und Methacrylsäure eines 98:2 Gew.-Verhältnisses zum Restyling von Haar.

## Revendications

**1.** Composition de traitement capillaire comprenant un adhésif acrylique sensible à la pression constitué d'un copolymère d'acrylate de butyle et d'acide méthacrylique de rapport pondéral 98/2.

**2.** Composition selon la revendication 1 qui comprend en outre un tensioactif de conditionnement cationique.

**3.** Composition selon l'une quelconque des revendications précédentes qui comprend en outre un émollient.

**4.** Composition selon l'une quelconque des revendications précédentes qui comprend en outre une silicone volatile.

**5.** Composition selon l'une quelconque des revendications précédentes qui est une composition sans rinçage.

**6.** Composition de traitement capillaire selon l'une quelconque des revendications précédentes qui comprend en outre un agent propulseur hydrocarboné.

**7.** Procédé de coiffage des cheveux qui comprend la mise en contact des cheveux avec une composition selon l'une quelconque des revendications précédentes.

**8.** Utilisation d'un adhésif acrylique sensible à la pression constitué d'un copolymère d'acrylate de butyle et d'acide méthacrylique de rapport pondéral 98/2 permettant de redessiner une coiffure.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5166276 A
- EP 408311 A
- EP 412707 A
- EP 412704 A
- WO 2007071308 A
- EP 818190 A
- US 2798053 A